# EUROPEAN PATENT SPECIFICATION

(11) **EP 4 281 079 B1**
(45) Date of publication and mention of the grant of the patent: **05.08.2026**
(21) Application number: 22704501.0
(22) Date of filing: 21.01.2022
(51) Int. Cl.: A61K 31/519, A61P 11/00, A61P 31/14

(54) **CILENGITIDE FOR USE IN A TREATMENT OF A CORONAVIRUS INFECTION**
CILENGITID ZUR VERWENDUNG IN DER BEHANDLUNG VON CORONAVIRUS-INFEKTION
CILENGITIDE POUR L'UTILISATION DANS LE TRAITEMENT D'UNE INFECTION À CORONAVIRUS

(30) Priority: 22.01.2021 EP 21153061
(43) Date of publication of application: 29.11.2023
(73) Proprietor: Royal College of Surgeons in Ireland, D2 Dublin (IE)
(72) Inventor: KERRIGAN, Steven, Dublin, Dublin 2 (IE); NADER, Danielle, Dublin, Dublin 2 (IE)
(74) Representative: Purdylucey Intellectual Property
(86) International application number: PCT/EP2022/051399
(87) International publication number: WO 2022/157336

(56) References cited:
- EP-A1- 3 207 937
- US-A1- 2007 155 750
- US-A1- 2020 289 534
- B\'ALINT M\'ESZ\'AROS ET AL: "Short linear motif candidates in the cell entry system used by SARS-CoV-2 and their potential therapeutic implications", ARXIV.ORG, CORNELL UNIVERSITY LIBRARY, 201 OLIN LIBRARY CORNELL UNIVERSITY ITHACA, NY 14853, 21 April 2020 (2020-04-21), XP081650097

## Description

### Field of the invention

The current invention relates to a therapeutic strategy for the treatment and prevention of coronavirus. In particular, the invention relates to an αVβ3 integrin antagonist for use in a method of treatment or prevention of a disease caused by a coronavirus, more specifically, Coronavirus disease 2019 (COVID-19).

### Background of the invention

The unprecedented and ongoing Coronavirus disease 2019 (COVID-19) pandemic has caused a severe shock to the entire global population. As the global death toll continues to rise, vaccine candidates, and targeted effective anti-viral and immunotherapies are desperately sought after.

Compared with diseases from other coronaviruses, COVID-19 has more adverse effects on the cardiovascular system. High rates of deep vein thrombosis and pulmonary embolism have been associated with severe SARS-CoV-2 infection (Klok FA, et al. Incidence of thrombotic complications in critically ill ICU patients with COVID-19. Thromb Res. 2020; 191:145-7) while COVID-19 presentations with stroke, myocardial infarction and disseminated intravascular coagulopathy have been reported (Oxley TJ, et al. Large-Vessel Stroke as a Presenting Feature of Covid-19 in the Young. N Engl J Med. 2020;382(20):e60). In addition, accumulating evidence suggests that microvascular occlusion within the lungs plays an important role in COVID-19 pathogenesis. Post-mortem studies have demonstrated widespread microthrombi throughout the pulmonary vasculature in patients with fatal COVID-19 (Wichmann D, et al. Autopsy Findings and Venous Thromboembolism in Patients With COVID-19: A Prospective Cohort Study. Ann Intern Med. 2020;173(4):268-77), and right ventricular dysfunction in critical ill COVID-19 patients is common (Giustino G, et al. Characterization of Myocardial Injury in Patients With COVID-19. J Am Coll Cardiol. 2020;76(18):2043-55). Data from autopsy studies in COVID-19 has identified marked endothelial cell apoptosis, together with loss of tight junction integrity in the pulmonary microvasculature, while electron microscopy studies have shown SARS-CoV-2 viral particles within pulmonary endothelial cell, suggesting that direct pulmonary endothelial cell infection may be important in triggering COVID-19 associated vasculopathy (Varga Z, et al. Endothelial cell infection and endotheliitis in COVID-19. Lancet. 2020;395(10234):1417-8).

Vascular endothelial cells are essential in viral propagation, yet the mechanistic understanding behind this pathway remains elusive. The etiological pathogen of COVID-19, severe acute respiratory syndrome coronavirus 2 (SARS-CoV-2), is far more transmissible than its coronavirus predecessors. It is clear that SARS-CoV-2 possesses a new characteristic not shared with previous coronaviruses which has resulted in a widespread pandemic. Studies have established that the SARS-CoV-2 spike protein is significantly involved in host recognition and viral attachment and interacts with the host receptor angiotensin-converting enzyme 2 (ACE2) through its receptor binding domain.

However, a novel K403R mutation in the spike protein of SAR-CoV-2 forms a unique Arg-Gly-Asp (RGD) motif outside the ACE2 recognition site.

The endothelial barrier is highly populated with integrins, where the major endothelial cell integrin, αVβ3, is capable of recognising a plethora of RGD-containing ligands. It contains a binding pocket that interacts with various proteins across the extracellular matrix, including fibrinogen, fibronectin, and vitronectin, which function to regulate adhesion, cellular migration, proliferation and angiogenesis.

US2020/0289534 describes a method of treating, reducing, or alleviating a medical condition, including administering a biocompatible drug comprising one or more antiviral medications. Administered with this medication is one or more cell pathway inhibitors dissolved in a non-toxic semi fluorinated alkane. The listed examples of medical conditions include one or more respiratory tract inflammatory disease. The inhibitors are stated to block an inflammatory response of inflamed tissue and the alkane evaporating upon administration so as to leave the biocompatible drug at a desired treatment location. There is one example discussing coronavirus and prevention, in which the subject was treated prophylactically with application of oseltamivir, baricitinib and a GSK inhibitor and a COVID-19 S timer intranasally followed with deep breathing 3-4 times daily in a semi fluorinated alkane for 3 days. The subject returned from travels without signs of coronavirus. There are no data or further results presented regarding this example. Although this publication lists cilengitide as one of the many cell pathway inhibitors, there are no data using αVβ3 integrin for any respiratory indication nor are there any data using cilengitide for any indication, such as coronavirus.

EP3207937 describes a functional αVβ3 antagonist, such as cilengitide, for the treatment or prevention of sepsis in a patient

US2007/155750 relates, in general, to integrin-binding small molecules. More specifically, the authors describe compositions and methods of using these compositions for treating various diseases. The application describes the therapeutic potential of αVβ3 antagonists.

The current invention provides a therapeutic strategy for the treatment and prevention of a disease caused by a coronavirus comprising an αVβ3 integrin antagonist.

### Summary of the invention

The current inventors have surprisingly shown that the SARS-CoV-2 spike protein exploits human endothelial αVβ3 integrin to mediate its attachment to the host, through a novel K403R mutation in its receptor binding domain. This has not been shown previously in the art.

By developing this evolutionary mutation, i.e., K403R, which provides the spike protein with an additional binding site, RGD, the inventors suggest that could increase SARS-CoV-2 pathogenicity by enhancing viral attachment to host and causing higher transmissibility rates. Additionally, both ACE2 and αVβ3 integrin receptors are abundantly present across the human vasculature, providing several possible routes of entry for the virus whilst promoting dissemination across the host through a dual-receptor mechanism.

The inventors have demonstrated that upon binding to the human vasculature, via αVβ3, SARS-CoV-2 causes significant endothelial dysregulation resulting in loss of barrier function/ integrity promoting shock and dissemination of secondary infection to major organs. This injurious effect inflicted by the virus could be significantly reduced by preventing the interaction with the major endothelial cell integrin αVβ3. This has not been previously disclosed or predicted by the prior art.

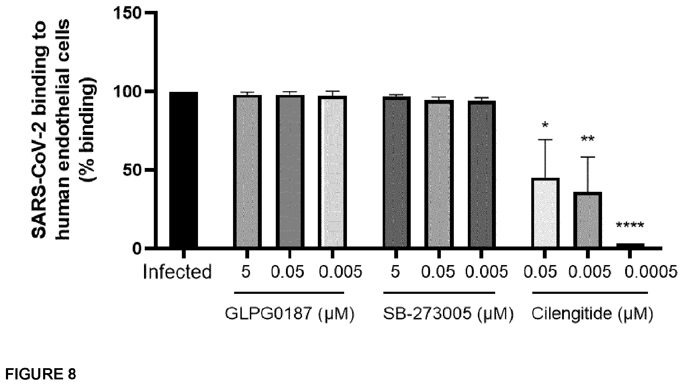

Furthermore, these data suggest that cilengitide is behaving in a different unexpected beneficial way in preventing viral attachment to human vascular endothelial cells in comparison to other integrin antagonists (Figure 8).

The invention is as set out in the appended claims.

In a first aspect, the current invention provides an αVβ3 integrin antagonist cilengitide("αVβ3 integrin antagonist of the invention") for use in a method of treatment or prevention of a coronavirus infection in a subject.

In an embodiment, the coronavirus is one comprising a K403R mutation in the receptor binding domain of the spike protein, providing an RGD domain.

Preferably, the coronavirus is severe acute respiratory syndrome-related coronavirus (SARS-CoV).

Preferably, the coronavirus is SARS-CoV-2 and the subject has the disease or condition COVID-19.

In an embodiment, the αVβ3 antagonist is formulated as a composition comprising the antagonist. The composition may be formulated for oral or intravenous administration. The composition may be formulated for pulmonary administration.

The composition may be a pharmaceutical composition. Such a composition may comprise one or more pharmaceutically acceptable carrier or excipient.

The references to the method of treatment or prevention of a coronavirus infection are to be interpreted as references to compounds, pharmaceutical compositions and medicaments of the present invention for use in those methods.

A method of treatment or prevention of a coronavirus infection is provided, said method comprising administration of an αVβ3 antagonist to a subject. The αVβ3 integrin antagonist is that as described herein. The coronavirus is as described herein.

### Brief Description of the Figures

The invention will be more clearly understood from the following description of an embodiment thereof, given by way of example only, with reference to the accompanying drawings, in which:

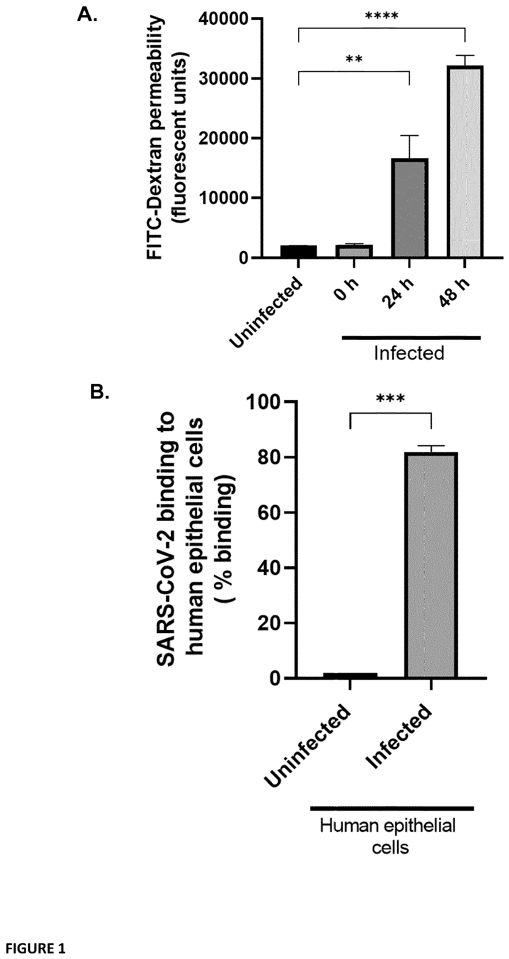

**Figure 1** **(A and B):** SARS-CoV-2 binds to human epithelial cells and causes permeability. (A) Human colonic epithelial cell line Caco-2 allowed to adhere onto immobilised SARS-CoV-2 at MOI=0.4. Cells were allowed to adhere to immobilised SARS-CoV-2 and lysed with pNPP, a fluorescent substrate against alkaline phosphatase expressed within cells. The fluorescent signal emitted by pNPP correlated to the number of cells adhered and was read at 405 nm. Epithelial cells significantly interacted with SARS-CoV-2 (P<0.001; paired t-test, N=3). (B) Caco-2 was seeded onto transwell inserts and infected with SARS-CoV-2 at MOI=0.4. Permeability was measured using Fluorescein isothiocyanate-dextran (FITC-Dextran, 40kDa) across 0 hours, 24 hours, and 48 hours. FITC-Dextran passes through the epithelial cells into the lower chamber and is proportionate to their permeability. The extent of permeability was measured by quantifying the fluorescent levels of FITC-Dextran at 490/520 nm. Cell permeability significantly increased throughout the time course (P<0.01; P<0.0001; ANOVA, N=3).

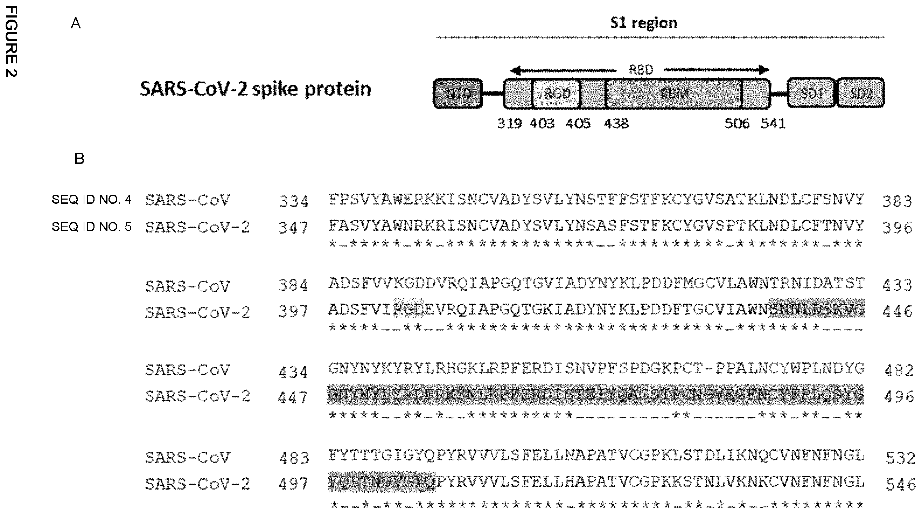

**Figure 2** **(A and B):** Sequence comparison between SARS-CoV and SARS-CoV-2 highlights the novel K403R mutagenesis. (A) Overall schematic drawing of the SARS-CoV-2 spike protein shows the N-terminal domain (NTD), RGD motif (Arg-Gly-Asp), receptor binding domain (RBD), receptor binding motif (RBM), sub-domain 1 (SD1), sub-domain 2 (SD2). The RGD motif resides within the receptor binding domain but adjacent due to the ACE2-binding region (RBM). (B) Pairwise sequence alignment using EMBL-EMBOSS Needle contrasts the spike protein of SARS-CoV and SARS-CoV-2. Identical amino acids are signified by (*), dissimilar amino acids are signified by (-), RGD motif (yellow), RBM region (blue).

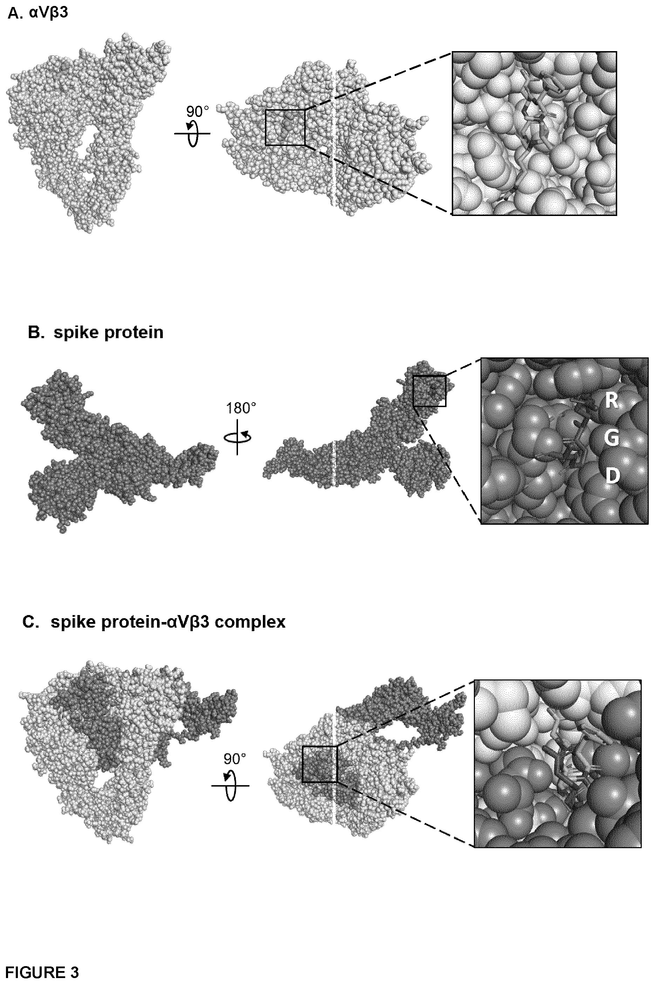

**Figure 3** **(A to C):** In-silico predicted structure of SARS-CoV-2 spike protein in complex with αVβ3. Docking was performed to determine whether the RGD motif of SARS-CoV-2 binds the RGD-pocket of integrin αVβ3. (A) Integrin αVβ3 (PDB: IL5G) was visualised (yellow) with a side panel highlighting the RGD ligand (blue). (B) Spike protein (PDB: 6VXX) was visualised (purple) with a side panel locating the novel RGD motif (pink). (C) Complex of spike protein-αVβ3 reveals the RGD motif of SARS-CoV-2 fits into the same binding pocket that αVβ3 forms to bind any RGD ligand. Protein structures were constructed using PyMol.

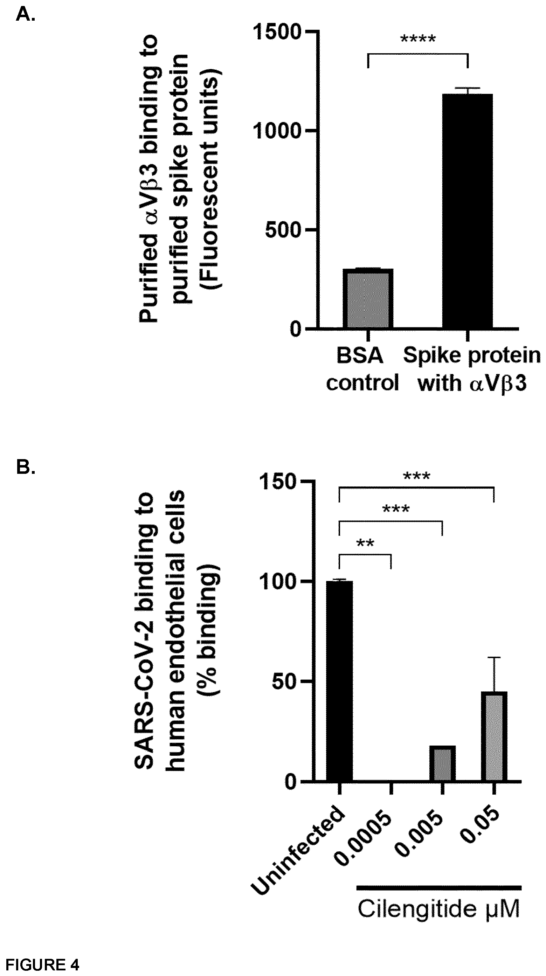

**Figure 4** **(A and B):** The RGD motif of SARS-CoV-2 spike protein mediates interaction to human endothelial cells. (A) The interaction between purified viral spike protein and endothelial integrin protein was assessed through an immunofluorescence binding assay. Recombinant SARS-CoV-2 spike protein was immobilised and its binding to recombinant alpha V beta 3 protein was measured using an anti-αVβ3 fluorescent antibody (LM609-AF488, 1:100). Binding was analysed by measuring absorbance at 450 nm. The spike protein bound to αVβ3 showed significant interaction (P<0.0001; paired t-test, N=3). (B) An in-vitro infection model investigated the adhesion potential of SARS-CoV-2 to endothelial cells. Sheared human aortic endothelial cells were activated with the cytokine TNFα to induce a pro-inflammatory state similar to that experienced in COVID-19 sepsis. Cilengitide was administered in 10-fold increments (0.05 µM - 0.0005 µM). A pNPP binding assay indicated % binding according to fluorescent signal measured at 405 nm. Statistical significance was found between the no drug control and each Cilengitide treated group. Since 0.0005 µM eliminated the host-viral interaction, it was chosen as optimal concentration for subsequent experiments (P=0.001, ANOVA, N=3).

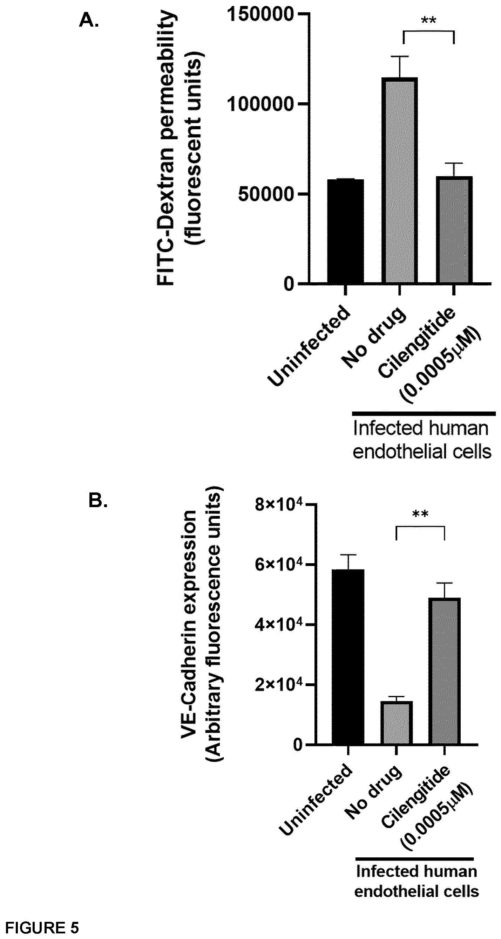

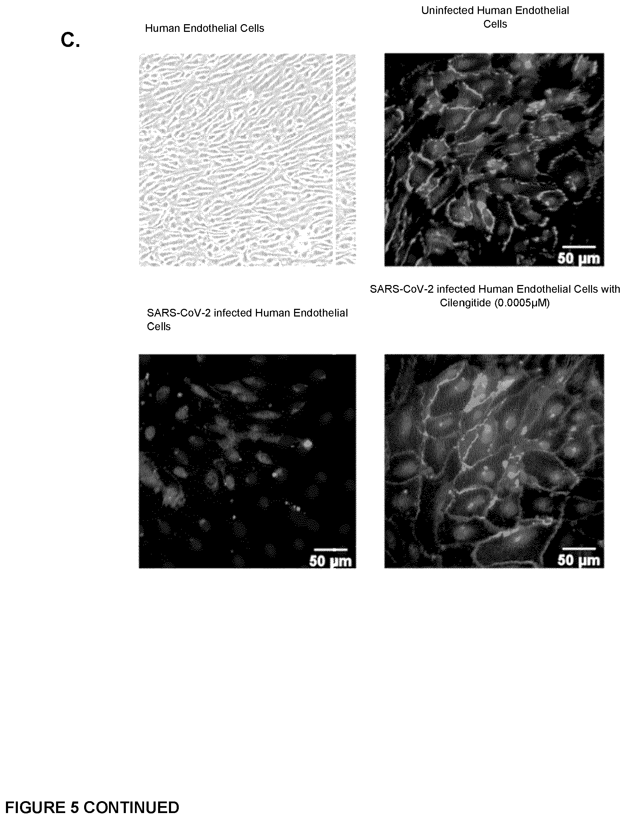

**Figure 5** **(A to C):** Vascular dysregulation occurs during SARS-CoV-2 infection and can be prevented using an αVβ3 antagonist, Cilengitide. (A) Both treated and untreated endothelial cells were seeded, sheared, and activated in transwell inserts to simulate the physiological conditions of blood flow and stress experienced by the vasculature in-vivo. Following Cilengitide treatment (0.0005 µM), cells were inoculated with SARS-CoV-2 for 24 hours. Fluorescein isothiocyanate-dextran (FITC-Dextran, 40kDa) was added, which passed through the endothelial cell monolayer into the lower chamber, proportionate to the barrier's permeability. The extent of permeability was measured by quantifying the fluorescent levels of FITC-Dextran at 490/520 nm. The significant rise in permeability levels indicated severe loss of barrier integrity following SARS-CoV-2 infection but was restored after treating cells with Cilengitide (P=0.005; ANOVA, N=3). (B) Vascular-endothelial cadherin (VE-Cadherin) expression on sheared, activated endothelial cells was assessed using immunofluorescence microscopy. Treated cells received 0.0005 µM Cilengitide. Infected cells were inoculated with SARS-CoV-2 for 24 hours. Cell junctions were stained with anti-VE Cadherin antibody (green) and 4,6-diamidino-2-phenylindole (blue) for nuclei staining. Scale bar at 50 µm. Uninfected cells were first visualised using inverted phase-contrast microscopy at magnification 20X to observe shear connections (top left) and compared to uninfected cells using fluorescent microscopy at magnification 43X (top right). Infected endothelial cells experienced loss of monolayer connections, by reduced expressed of VE-Cadherin (bottom left). Blocking the viral RGD to host αVβ3 pathway with Cilengitide re-established the barrier integrity (bottom right). (C) VE-cadherin levels in uninfected, infected, and treated cells were assessed as a marker of barrier integrity and quantified based on mean fluorescence. The vasculature experienced severe vessel leakage following viral infection, due to significantly reduced expression of VE-Cadherin. When treated with Cilengitide, barrier permeability was restored back to uninfected levels (P=0.02; ANOVA, N=3).

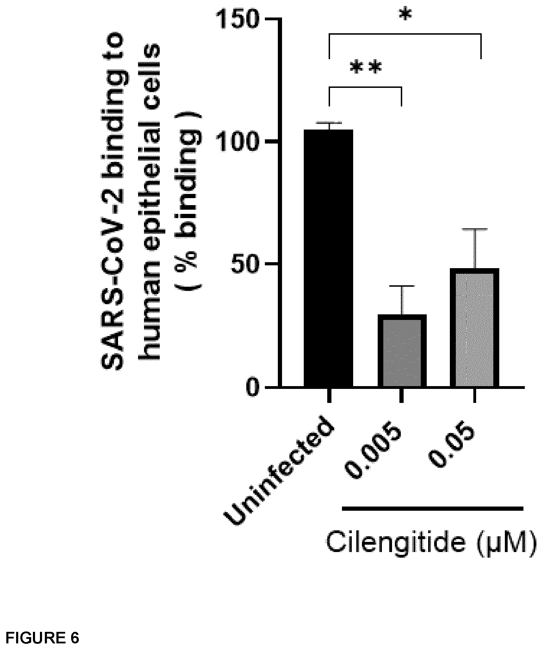

**Figure 6****:** Cilengitide effectively reduces SARS-CoV-2 binding to human colorectal epithelial cells in an *in vitro* infection model. P<0.05, **P<0.01.

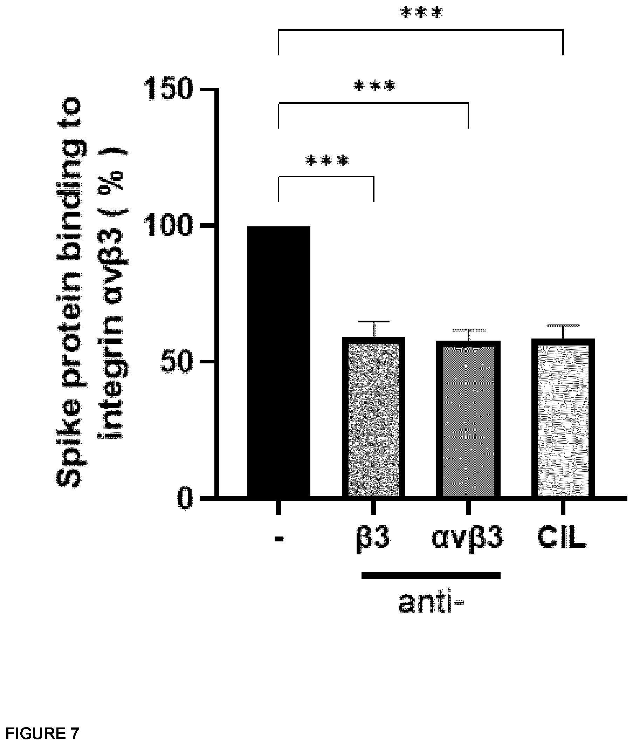

**Figure 7****:** Spike protein binding to purified integrin αVβ3. Both αVβ3 and β3 antibodies can inhibit spike protein binding to αVβ3, revealing significant interactions between the receptors. Cilengitide prevents binding between recombinant spike protein and αVβ3 at 0.0005 µM.***P<0.001.
**Figure 8****:** Comparison of non-RGD binding integrin antagonist compound GLPG0187 reveals Cilengitide is significantly more effective in reducing recombinant spike protein binding to integrin αVβ3. An *in vitro* infection assay using SB-273005, a non-RGD binding integrin antagonist, also was unsuccessful at reducing SARS-CoV-2 binding to human endothelial cells. *P<0.05, **P<0.01.

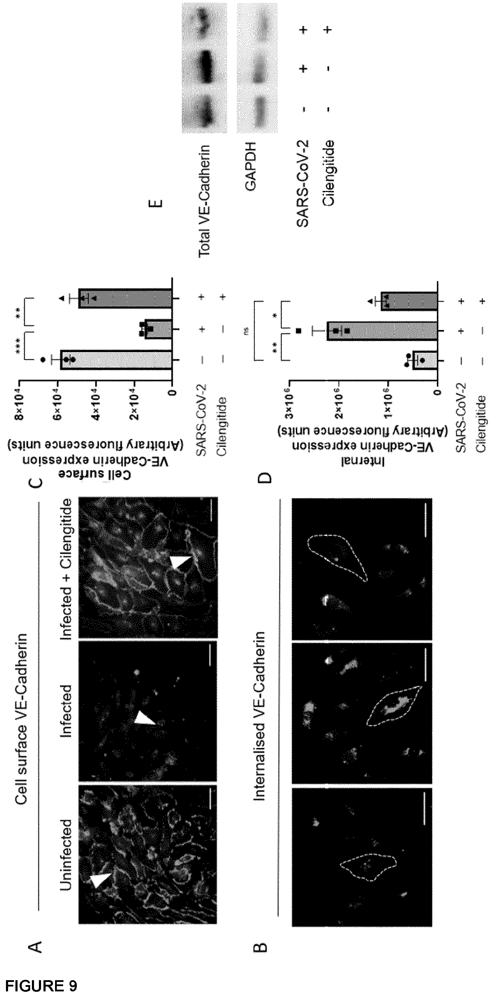

**Figure 9** **(A-E):** Following infection by SARS-CoV-2, the vascular endothelium experiences severe monolayer dysfunction, identified by VE-Cadherin relocation from the external cell-cell contacts to internal cellular compartments. The internalisation of VE-Cadherin is prevented in the presence of Cilengitide, in an in vitro model of infection (0.0005 µM). Total VE-Cadherin remains consistent in both healthy, infected, and treated cells, revealing VE-Cadherin translocation patterns. *P<0.05, **P<0.01, ***P<0.0001, NS=Not significant

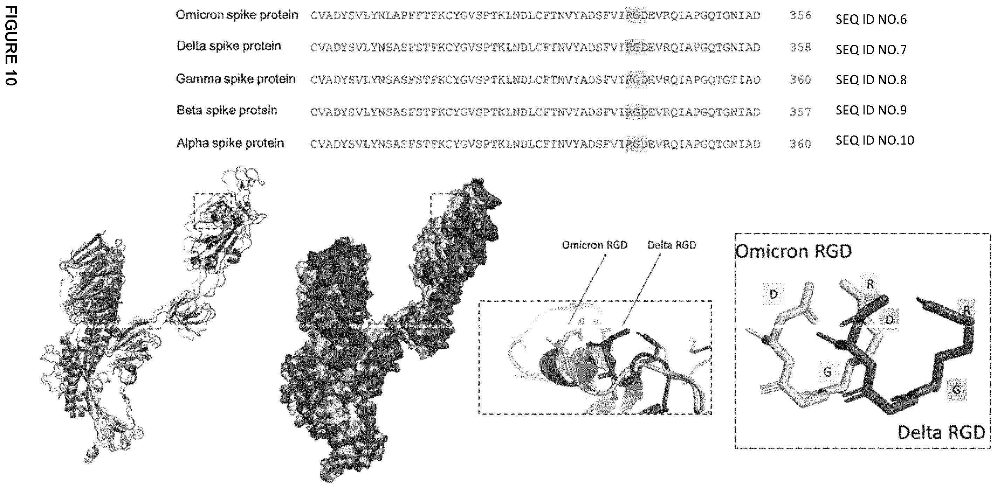

**Figure 10****:** SARS-CoV-2 variants of concern Alpha, Beta, Gamma, Delta and Omicron spike proteins aligned to show conservation of RGD site at 403-405. *In silico* modelling of Omicron and Delta spike proteins reveals RGD motif of Omicron variant extends outwards to the solvent more than Delta. Color key: Omicron (yellow), delta (pink).

### Detailed description of the invention

In its broadest sense, the current invention provides an αVβ3 integrin antagonist cilengitide for use in a method of treatment or prevention of a coronavirus infection in a subject. It may be for the treatment or prevention of a disease or condition caused by a coronavirus in a subject.

Typically, the coronavirus is one associated with loss of barrier function. Notably, the coronavirus is one associated with cardiovascular effects. Specifically, SARS-CoV-2 elicits an acute inflammatory effect with hypercoagulability, platelet activation, and endothelial dysfunction. Other cardiovascular effects or conditions include, but are not limited to, one or more of deep vein thrombosis, pulmonary embolism, stroke, myocardial infarction, disseminated intravascular coagulopathy, microvascular occlusion e.g., in the lungs, such as microthrombi in pulmonary vasculature and right ventricular dysfunction.

Notably, the coronavirus is SARS-CoV-2 and the disease caused by SARS-CoV-2 is COVID-19. The coronavirus is one with an RGD domain in the spike protein.

It may be any variant of SARS-CoV-2. Several variants of concern (one that causes severe disease in humans) of SARS-CoV-2 are now known, and any variant is encompassed herein. The variant also has an RGD domain in the spike protein. It may be a zoonotic variant or version of SARS-CoV-2. Examples include but are not limited to, SARS-CoV-2 cluster 5i.e., "mink variant", VOC 2020/21 i.e., "UK variant", 501Y.V2 i.e., "South African (SA) variant" and B.1.1.28 (E484K) i.e., "Brazilian variant". It may be the alpha variant, beta variant, gamma variant, delta variant or omicron variant. Such variants and identifiable in the art.

The current inventors have shown that all SARS-CoV-2 variants of concern Alpha, Beta, Gamma, Delta and Omicron spike proteins aligned to show conservation of RGD site at 403-405 (Figure 10).

The αVβ3 integrin antagonist cilengitide is a therapeutically effective amount of the antagonist.

Notably, the coronavirus is one with an RGD site or motif present in the amino acid sequence of the spike protein. The sequence of the spike protein is as follows: >QHD43416.1 surface glycoprotein [Severe acute respiratory syndrome coronavirus 2; SEQ ID NO. 3]

The RGD site is in bold, highlighted in yellow and underlined in the above sequence. The RGD site enables the virus to bind to αVβ3 integrin on endothelial cells in a subject. The endothelial cells are typically any vascular endothelial cells.

In an aspect, the antagonist of the invention cilengitide is used as a preventative to prevent, or reduce the severity, of coronavirus infection, e.g. SARS-CoV-2 infection, in a subject. In this context administration of αVβ3 integrin antagonist to the subject prevents the virus from attaching to the endothelial cells and causing endothelial dysfunction.

The subject may receive the antagonist at any time to prevent infection. The subject may receive the antagonist when the subject becomes aware of exposure to a known case of the coronavirus, or a suspected case of the disease, or when a subject is at risk of developing the disease. Administration should begin immediately, e.g., upon exposure or suspected exposure. Administration may be within one hour, or 12 hours, 24 to 48 hours, or within 7 days of exposure or suspected exposure.

In an aspect cilengitide is used to treat a coronavirus infection, for example, SARS-CoV-2 infection, in a subject. Administration of αVβ3 integrin antagonist to the subject prevents the virus from attaching and entering the subject's endothelial cells, for example the vascular endothelial cells, thus preventing vascular damage or further vascular damage. The virus can bind αVβ3 abluminally, opening up the endothelial cells and causing leakage and allowing all pathogens to disseminate into the bloodstream.

This treatment stops endothelial cell damage or dysregulation associated with binding of the virus which results in loss of barrier function. This is a key problem with the dissemination of the virus and opportunistic bacteria around the body and is the primary manifestation of the cardiovascular effects seen in coronavirus infection. This may cause sepsis.

VE-cadherin is a crucial tight junction protein that plays a key role in maintaining an intact endothelial layer and the inventors have found its surface expression on endothelial cells significantly reduced upon infection with SARS-CoV-2. The virus activates αVβ3 integrin, which results in VE-cadherin phosphorylation and internalisation. Cilengitide works by preventing VE-cadherin reduction during infection.

The subject may receive the treatment, or antagonist of the invention, at any time. The subject may receive the antagonist when the subject becomes aware of exposure to a known case of the coronavirus or a suspected case of the disease, or when a subject is at risk of developing the disease, or once the subject tests positive and/or is displaying symptoms of the disease. The treatment may be when a subject presents at hospital, within one hour, 12 hours, 24 to 48 hours, of presenting at hospital.

The "subject" may be any person. In an embodiment, the subject may be a person at risk of developing the disease caused by a coronavirus, such as COVID 19, for example, a close contact with known exposure to SARS-CoV-2. The subject may be at risk due to having been in contact, typically close contact, with a subject that has been diagnosed or that displays symptoms of the disease caused by a coronavirus, such as COVID-19. The subject may be one who is suspected of having a disease or infection of a coronavirus, e.g. COVID-19 or is displaying symptoms of said disease and/or has tested positive for said disease. The subject may of any age group. The subject may be a subject over 65 years old. The subject may be an immune compromised subject. The subject may be a care worker, including a hospital worker such as a doctor, a nurse or a carer.

The dose and frequency of the dose may be any suitable dose and frequency depending on the subject and/or the severity of the disease. In practicing the invention, the amount or dosage range of the αVβ3 integrin antagonist employed typically is one that effectively induces, promotes, or enhances a physiological response associated with αVβ3 integrin antagonist of the invention. In one aspect, the dosage range is selected such that αVβ3 integrin antagonist of the invention employed induces, promotes, or enhances a medially significant effect in a patient suffering from or being at substantial risk of developing coronavirus infection or a disease caused by a coronavirus.

In use, the dosage or amount may be administered once every day, or once every 2, 3, 4, 5, 6, 7 days. It may be a single or repeated dose. The dose may be administered every week, fortnight, month, two months, three months, or four months. It may be daily for a period of 2 to 14 days.

In use for treatment, it may be on at least one of day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, post infection or diagnosis, or alternatively, at least one of week 1, 2, 3, 4, 5, 6, 7, 8, 9, 10, 11, 12, 13, 14, 15, 16, 17, 18, 19 or 20, or any combination thereof, using single, continuous or divided doses of every or any combination thereof.

Treatment can be provided by administration of a daily dosage of αVβ3 integrin antagonist of the invention in an amount of about 0.19ng/kg or less to the subject.

In one embodiment, the therapeutically effective amount is less than 100mg/m². "mg/m²" means mg per m² of body surface area, and a method of calculating body surface area is provided at www.halls.md/body-surface-area/bsa.htm.

In one embodiment, the therapeutically effective amount is less than 50mg/m². In one embodiment, the therapeutically effective amount is less than 10mg/m². In one embodiment, the therapeutically effective amount is less than 5mg/m². In one embodiment, the therapeutically effective amount is less than 1mg/m². In one embodiment, the therapeutically effective amount is 0.001 to 50mg/m². In one embodiment the therapeutically effective amount is 0.001 to 10mg/m². In one embodiment, the therapeutically effective amount is 0.001 to 1.0mg/m². In one embodiment, the therapeutically effective amount is 0.01 to 10mg/m². In one embodiment, the therapeutically effective amount is 0.1 to 10mg/m². In one embodiment, the therapeutically effective amount is 0.001 to 1.0mg/m². In one embodiment, the therapeutically effective amount is 0.01 to 1mg/m². In one embodiment, the therapeutically effective amount is 0.1 to 1mg/m². In one embodiment, the therapeutically effective amount is administered once daily for a period 2 to 14 days.

In an embodiment, a dosage of 10mg/m² or less is administered to the patient. In an embodiment, a dosage of less than 10mg/m² is administered to the patient. In an embodiment, a dosage of 1mg/m² or less is administered to the subject. In an embodiment, a dosage of less than 1mg/m² is administered to the subject.

The inventors surprisingly found that there was an inverse dose response with the αVβ3 integrin antagonist of the invention, where the higher doses show attenuated effect or no effect at all. This is illustrated by Figure 4B. The antagonist elicited effects an inverted dose response pattern.

Also provided by the current invention is the αVβ3 integrin antagonist cilengitide for use in a method of treatment or prevention of coronavirus disease characterised by loss of barrier function in a subject.

Also provided by the current invention is cilengitide for use in a method of treatment or prevention of cardiovascular dysfunction associated with coronavirus infection in a subject. In one instance not forming a part of the invention, the cilengitide is for treating of cardiovascular effects associated with coronavirus infection and the effects as described herein comprise acute inflammatory effect with hypercoagulability, platelet activation, endothelial dysfunction, deep vein thrombosis, pulmonary embolism, stroke, myocardial infarction, disseminated intravascular coagulopathy, microvascular occlusion e.g., in the lungs, such as microthrombi in pulmonary vasculature and right ventricular dysfunction.

The coronavirus is as described herein.

It will be appreciated that the embodiments or preferred features described in relation to the medical use of the invention also apply to the methods of treatment or prevention of the invention.

### Definitions and general preferences:

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g., a feature, element, characteristic, property, method/process step or limitation) or group of integers (e.g., features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition, or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

In this context the "disease" to be treated or prevented is a coronavirus, in particular a coronavirus characterised by loss of barrier function. The coronavirus may be a severe acute respiratory syndrome-related coronavirus (SARS-CoV), for example, SARS-CoV-2 (COVID-19).

As used herein, the term "treatment" or "treating" refer to an intervention (e.g., the administration of an agent to a subject) which cures, ameliorates, or lessens the symptoms of a disease or removes (or lessens the impact of) its cause(s). In this case, the term is used synonymously with the term "therapy". It can be manifested by a permanent or temporary improvement in the subject's condition. In this case, treatment can include an initial step of prevention or prophylaxis.

As used herein the terms "prevention" or "preventing" refer to an intervention (e.g. the administration of an agent to a subject), which prevents or delays the onset or progression of a disease, e.g. COVID-19 and/or the severity of a disease, e.g. COVID-19, in a subject, or reduces (or eradicates) its incidence within a treated population, and/or prevents or delays the entry of a virus, e.g. SARS-CoV-2, into the cells of a subject, In this case, the term treatment is used synonymously with the term "prophylaxis".

As used herein, the term "effective amount or a therapeutically effective amount" as applied to the αVβ3 antagonist, defines an amount that can be administered to a subject without excessive toxicity, irritation, allergic response, or other problem or complication, commensurate with a reasonable benefit/risk ratio, but one that is sufficient to provide the desired effect, e.g., prevent binding of a coronavirus to a receptor, in an individual. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes the reduction in the amount of a virus, such as SARS-CoV-2, infecting cells of a subject and/or slowing or inhibiting the occurrence of respiratory failure, in an individual. A therapeutic result need not be a complete cure. A therapeutic result may be a permanent or temporary improvement in the subject's condition Amount can be used interchangeably with dose.

In the context of treatment and effective amounts as defined above, the term "individual" (which is to be read to include "subject", "animal", "patient" or "mammal" where context permits) defines any individual, particularly a mammalian individual, for whom treatment is indicated. Mammalian subjects include, but are not limited to, humans, domestic animals, farm animals, zoo animals, sport animals, pet animals such as dogs, cats, guinea pigs, rabbits, rats, 5 mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

"αVβ3" is a member of the integrin supergene family of cell surface glycoprotein receptors that promote cellular adhesion. It binds to a variety of plasma and extracellular matrix proteins containing the conserved RGD amino acid sequence and modulates cell adhesion. It is highly expressed in osteoclasts where it plays a role in bone resorption and is also abundant in vascular smooth muscle cells and endothelial cells, and in some tumour cells, where it is involved in angiogenesis and cell migration (Felding et al, Curr Opin Cell Biol. 1993;5;864-868). "vascular endothelial αVβ3" refers to the αVβ3 integrin expressed by vascular endothelial cells.

"αV domain" refers to the αV domain of the vascular endothelial αVβ3 integrin. The sequence of human vascular endothelial αV domain is provided in SEQUENCE ID NO: 1:
*ITAV_HUMAN Integrin alpha-V OS=Homo sapiens (SEQUENCE ID NO: 1)*

MAFPPRRRLRLGPRGLPLLLSGLLLPLCRAFNLDVDSPAEYSGPEGSYFGFAVDFFVPSAS
SRMFLLVGAPKANTTQPGIVEGGQVLKCDWSSTRRCQPIEFDATGNRDYAKDDPLEFKSH
QWFGASVRSKQDKILACAPLYHWRTEMKQEREPVGTCFLQDGTKTVEYAPCRSQDIDADG
QGFCQGGFSIDFTKADRVLLGGPGSFYWQGQLISDQVAEIVSKYDPNVYSIKYNNQLATRT
AQAIFDDSYLGYSVAVGDFNGDGIDDFVSGVPRAARTLGMVYIYDGKNMSSLYNFTGEQM
AAYFGFSVAATDINGDDYADVFIGAPLFMDRGSDGKLQEVGQVSVSLQRASGDFQTTKLN
GFEVFARFGSAIAPLGDLDQDGFNDIAIAAPYGGEDKKGIVYIFNGRSTGLNAVPSQILEGQ
WAARSMPPSFGYSMKGATDIDKNGYPDLIVGAFGVDRAILYRARPVITVNAGLEVYPSILNQ
DNKTCSLPGTALKVSCFNVRFCLKADGKGVLPRKLNFQVELLLDKLKQKGAIRRALFLYSRS
PSHSKNMTISRGGLMQCEELIAYLRDESEFRDKLTPITIFMEYRLDYRTAADTTGLQPILNQF
TPANISRQAHILLDCGEDNVCKPKLEVSVDSDQKKIYIGDDNPLTLIVKAQNQGEGAYEAELI
VSIPLQADFIGVVRNNEALARLSCAFKTENQTRQVVCDLGNPMKAGTQLLAGLRFSVHQQS
EMDTSVKFDLQIQSSNLFDKVSPVVSHKVDLAVLAAVEIRGVSSPDHVFLPIPNWEHKENPE
TEEDVGPVVQHIYELRNNGPSSFSKAMLHLQWPYKYNNNTLLYILHYDIDGPMNCTSDMEI
NPLRIKISSLQTTEKNDTVAGQGERDHLITKRDLALSEGDIHTLGCGVAQCLKIVCQVGRLDR
GKSAILYVKSLLWTETFMNKENQNHSYSLKSSASFNVIEFPYKNLPIEDITNSTLVTTNVTWG
IQPAPMPVPVWVIILAVLAGLLLLAVLVFVMYRMGFFKRVRPPQEEQEREQLQPHENGEGN
SET

"β3 domain" refers to the β3 domain of the vascular endothelial αVβ3 integrin. The sequence of human vascular endothelial β3 domain is provided in SEQUENCE ID NO: 2:
*ITB3_HUMAN Integrin beta-3 OS=Homo sapiens (SEQUENCE ID NO: 2)*

The term "αVβ3 antagonist" refers to a compound or molecule that bind to a αVβ3 integrin without activating the receptor itself. Examples include peptides {cyclic and noncyclic}, non-peptide ligands, disintegrins, peptidomimetics, antibodies, and gene inhibitors. An example of a cyclic peptide is cilengitide. Examples of antibody antagonists are described in the literature, for example US5652110. αVβ3 antagonists are also described in Millard et al {Theranostics 2011; 1:154=188}, Kim et al (Mol Pharm 2013 Oct 7, 3603-3611), Pfaff et al (J Biol Chem 1994; 269, 20233-20238), Healy et al (Biochemistry 1995; 34, 3948-3955), EP2298308 (University of Southern California), Koivunen et al (J Biol Chem 1993;268; 20205-20210 and 1994;124:373-380), Ruoslahti et al (Annu Rev Cell Dev Biol. 1996; 12:697-715), and Ponce et al (Faseb J. 2001;15;1389-1397). In one embodiment, the αVβ3 antagonist is selective for αV integrins.

In this context, preferably, the antagonist is one that binds to the endothelial αVβ3 integrin and prevents, or causes a decrease, in binding to coronavirus to the host integrin. In particular, the antagonist is one that binds to vascular endothelial cells. These include but are not limited to human aortic endothelial cells, microvascular endothelial cell, and human umbilical vein endothelial cell. The antagonist is one that binds αVβ3 integrin abluminally. *In vitro* binding assays are known in the art and described below. Examples include cilengitide and variants thereof described in EP0770622 (incorporated by reference). It is a routine matter for a skilled person to test the αVβ3 antagonists described in the literature using the in-vitro adhesion assay described below to identify functional αVβ3 antagonists.

"Cilengitide" is a potent integrin inhibitor for the αVβ3 receptor. It is a cyclic peptapeptide (cyclo(-RGDf(*N*Me)V-) which is selective for αV integrins (Jonczyk et al, European Patent Application No: 0770622). The synthesis and activity of the molecule was published in 1999 (Dechantsreiter et al J Med Chem 1999 Aug 12; 42(16)). Cilengitide has been indicated for treatment of cancer (EP2578225, EP2338518, EP2441464; incorporated by reference).

"Functional variant of Cilengitide" means the variants of cilengitide described in US6001961 which are integrin inhibitors for the αVβ3 receptor, specifically the cyclic peptides described in Sequence ID NO's 1 to 40 (US6001961, columns 12-13; incorporated by reference). A "functional variant" is a variant that has the same function, e.g., a variant of cilengitide which differs by a modification but maintains the same function, i.e. integrin inhibitor. Typically, the functional variant of cilengitide is a cyclic peptide comprising an RGD sequence or motif.

"Low molecular weight antagonist" means an antagonist that has a molecular of less than 50 KDa. In one embodiment, the low molecular weight antagonist has a molecular weight of less than 20 KDa. In one embodiment, the low molecular weight antagonist has a molecular weight of less than 10 KDa. In one embodiment, the low molecular weight antagonist has a molecular weight of less than 5 KDa. In one embodiment, the low molecular weight antagonist has a molecular weight of less than 4 KDa. In one embodiment, the low molecular weight antagonist has a molecular weight of less than 3 KDa. In one embodiment, the low molecular weight antagonist has a molecular weight of less than 2 KDa. In one embodiment, the low molecular weight antagonist has a molecular weight of less than 1 KDa.

"αVβ3 antibody" means an antibody that binds to the αVβ3 integrin. In one embodiment, the antibody binds specifically to the αVβ3 or αVβ5 integrin. In one embodiment, the antibody binds specifically to the αVβ3 integrin. Examples of αVβ3 antibodies are described in the literature (Liu et al Drug Dev Res 2008; 69(6) 329-339) and are commercially available, for example: MAB1976, a mouse monoclonal antibody commercially available from MerckMillipore (Clone LM609), also known as AVASTIN; Vitaxin I, a humanised monoclonal antibody (Coleman et al, Circulation Research 1999; 84; 1268-1276); ABEGRIN, a derivative of Vitaxin I manufactured by Medimmune; CNTO95, a fully humanised antibody that recognises multiple αV integrins and binds to αVβ3 or αVβ5 integrins with high affinity (Trika et al. Int J Cancer 2004;110; 326-335); c7E3 Fab (ABCIXIMAB or REOPRO) and c7E3(Fab')2, mouse-human chimeric and murine mAb fragments of the parent intact murine mAb 7E3 (Trikha et al. Cancer Res 2002;62;2824-2833 and Varner et al. Angiogenesis 1999;3;53-60); 17E6 antibody (Mitjans et al J Cell Sci 1995; 108; 2825-2838; The term "αVβ3 antibody" includes antibodies that selectively bind to the αV domain, the β3 domain, or both the αV and β3 domains. The term "functional αVβ3 antibody" means an αVβ3 antibody that binds to the vascular endothelial αVβ3 integrin and is capable of at least partially blocking adhesion of *S. aureus* or *E.coli* bacteria to endothelial cells in the *in-vitro* adhesion assay described below.

"Disintegrin" means the family of low molecular weight RGD containing cysteine rich peptides derived from viper venoms that can bind to αVβ3 and block its function. Examples include Trigramin, Kistrin, Bitistatin, Barbourin, Echistatin, Contortrostatin, all described or referenced in Liu et al Drug Dev Res 2008; 69(6) 329-339.

"Peptidomimetics" means compounds containing non-peptidic structural elements that are capable of mimicking the biological actions of peptidic αVβ3 antagonists. Examples include SC-68448 and SCH221153 both described or referenced in Liu et al Drug Dev Res 2008; 69(6) 329-339, and IS201 described in Bello et al (Neurosurgery 2003:52; 177186).

"Gene inhibitors" means a nucleic acid that can cause reduced expression of αVβ3. In some embodiments, the agent is a nucleic acid. A nucleic acid agent can be selected from, for example, siRNA, shRNA, miRNA, anti-microRNA, antisense RNA or oligonucleotide, aptamer, ribozyme, and any combinations thereof. When the agent is a nucleic acid, the agent itself can be administered to the subject or a vector expressing/encoding the agent can be administered to the subject. In some embodiments, the vector expressing/encoding the agent is an Adeno-associated virus (AAV) vector.

To practice the methods of this invention, the αVβ3 antagonist cilengitide can be administered orally, parenterally, by inhalation spray, topically, rectally, nasally, bucally, vaginally or via an implanted reservoir. The αVβ3 antagonist may be delivered by any form of pulmonary delivery. Such delivery means include but are not limited to metered dose inhalers, nebulizers and inhalers, e.g. dry powder inhalers. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. A sterile injectable composition, e.g., a sterile injectable aqueous or oleaginous suspension, can be formulated according to techniques known in the art using suitable dispersing or wetting agents (such as Tween 80) and suspending agents. The sterile injectable preparation can also be a sterile injectable solution or suspension in a non-toxic parentally acceptable diluents or solvent for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (eg. Synthetic mono-or dyglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluents or dispersant, or carboxymethyl cellulose or similar dispersing agents. Other commonly used surfactants such as Tweens or Spans or other similar emulsifying agents or bioavailablity enhancers which are commonly used in the manufacture of pharmaceutically acceptable solid, liquid, or other dosage forms can also be used for the purposes of formulation. Preferably, administration is oral administration. Typically, administration is intravenous. Typically, administration is pulmonary delivery.

A composition for oral administration can be any orally acceptable dosage form including, but not limited to, capsules, tablets, emulsions and aqueous suspensions, dispersions and solutions. It may be incorporated with excipients and used in the form of ingestible tablets, buccal tablets, troches, capsules, elixirs, suspensions, syrups, wafers, and the like, a hard or soft shell gelatin capsule or compressed into tablets. It may be coated, or co-administered with, a material to prevent its inactivation. In another aspect, the antagonist or composition of the invention may be orally administered, for example, with an inert diluent or an assimilable edible carrier.

In the case of tablets for oral use, carriers that are commonly used include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavouring, or colouring agents can be added. A nasal aerosol or inhalation composition can be prepared according to techniques well known in the art of pharmaceutical formulation. A fused multicyclic compound-containing composition can also be administered in the form of suppositories for rectal administration. The carrier in the pharmaceutical composition must be "acceptable" in the sense of being compatible with the active ingredient of the formulation (and preferable, capable of stabilising it) and not deleterious to the subject to be treated. For example, one or more solubilising agents, which form more soluble complexes with the fused multicyclic compounds, or more solubilising agents, can be utilised as pharmaceutical carriers for delivery of the active compounds. Examples of other carriers include colloidal silicon dioxide, magnesium stearate, sodium lauryl sulphate, and D&C Yellow #10.

In some embodiments of the current invention, administration or delivery may be via any one of liposomes, mixed liposomes, oleosomes, niosomes, ethosomes, millicapsules, capsules, macrocapsules, nanocapsules, nanostructured lipid carriers, sponges, cyclodextrins, vesicles, micelles, mixed micelles of surfactants, surfactant-phospholipid mixed micelles, millispheres, spheres, lipospheres, particles, nanospheres, nanoparticles, milliparticles, solid nanopartciles as well as microemulsions including water-in-oil microemulsions with an internal structure of reverse micelle and nanoemulsions microspheres, microparticles.

These delivery systems may be adapted to achieve a greater penetration of the antagonist or composition of the invention. This may improve pharmacokinetic and pharmacodynamic properties. The delivery system may be a sustained release system wherein the compound or peptide of the invention is gradually released during a period of time and preferably with a constant release rate over a period of time. The delivery systems are prepared by methods known in the art. The amount of the active contained in the sustained release system will depend on where the composition is to be delivered and the duration of the release as well as the type of the condition, disease and/or disorder to be treated or cared for.

"Antibody" refers to an intact αVβ3-binding immunoglobulin in any form, including but not limited to monoclonal, polyclonal, humanized or human form, or antibody fragments that bind to αVβ3. The term includes monoclonal or polyclonal αVβ3-binding fragments with the Fc (crystallizable fragment) region or FcRn binding fragment of the Fc region, referred to herein as the "Fc fragment" or "Fc domain". In one embodiment, the antibody has the Fc region removed or modified such that it cannot interact with Protein A of bacteria. Methods of making such antibodies are described in Hay et al (Immunochemistry 12(5):373-378), Parkham et al (J. Immunol 1983:131(6):2895-2902) and Baldwin et al (J. Immunol Methods 1989 121(2): 209-217). αVβ3-binding fragments may be produced by recombinant DNA techniques or by enzymatic or chemical cleavage of intact antibodies. αVβ3-binding fragments include, inter alia, Fab, Fab', F(ab')2, Fv, dAb, and complementarity determining region (CDR) fragments, single-chain antibodies (scFv), single domain antibodies, chimeric antibodies, diabodies and polypeptides that contain at least a portion of an immunoglobulin that is sufficient to confer specific antigen binding to the polypeptide. The Fc domain includes portions of two heavy chains contributing to two or three classes of the antibody. The Fc domain may be produced by recombinant DNA techniques or by enzymatic (e.g. papain cleavage) or via chemical cleavage of intact antibodies.

The antibody may be an IgG, an IgM, an IgE, an IgA or an IgD molecule. In a preferred embodiment, the antibody molecule is an IgG, and more preferably is of the IgG1, IgG2, IgG3, or IgG4 subtype. The class and subclass of antibodies may be determined by any method known in the art, for example, by using antibodies that are specific for a particular class and subclass of antibody. Antibodies specific to αVβ3 are available commercially and are described above. The class and subclass can be determined by ELISA and Western Blots, as well as other techniques. Alternatively, the class and subclass may be determined by sequencing all or a portion of the constant domains of the heavy and/or light chains of the antibodies, comparing their amino acid sequences to the known amino acid sequences of various class and subclasses of immunoglobulins, and determining the class and subclass of the antibodies.

Antibodies are a major class of biopharmaceuticals. Antibodies for therapeutic purposes are often produced from a cell line (e.g. CHO cells, the hamster line BHK21, the human PER.C6 cell line, COS, NIH 3T3, BHK, HEK, 293, L929, MEL, JEG-3, murine lymphoid cells (including NS0 and Sp2/0-Ag 14)), including hybridomas, and are usually a single clone of a specific antibody. Antibodies used for therapeutic purposes are classified as murine, chimeric, humanized or fully human antibodies and are produced by recombinant methods. A "murine antibody" is a full mouse antibody and has only limited use in humane due to its short half-life in circulation and its high immunogenicity. A "chimeric antibody" is a genetically engineered antibody, which contains both mouse and human sequences. The ratio is approximately 33% mouse contribution and 67 % human contribution. Usually the variable domains are murine and the constant region including the Fc fragment is derived from a human IgG.

A "humanized antibody" is a genetically engineered antibody, wherein the mouse content is reduced to about 5-10%. In such cases, the six CDRs of the heavy and light chains and a limited number of structural amino acids of the murine monoclonal antibody are grafted by recombinant technology to the CDR-depleted human IgG scaffold. A fully human antibody or human antibody describes antibodies, which are made in humanized mice resulting in antibodies that do not contain any mouse sequences, or made in vitro using phage libraries or ribosome display or alternatively are obtained from human donors. In certain embodiments, chimeric, humanized or primatized (CDR-grafted) antibodies, comprising portions derived from different species or fully human antibodies, are also encompassed by the present invention. The various portions of these antibodies can be joined together chemically by conventional techniques or can be prepared as a contiguous protein using genetic engineering techniques. For example, nucleic acids encoding a chimeric or humanized chain can be expressed to produce a contiguous protein. See, e.g., Cabilly et al., U.S. Pat. No. 4,816,567; Cabilly et al., European Patent No. 0,125,023; Boss et al., U.S. Pat. No. 4,816,397; Boss et al., European Patent No. 0,120,694; Neuberger, M. S. et al., WO 86/01533; Neuberger, M. S. et al., European Patent No. 0,194,276 B1; Winter, U.S. Pat. No. 5,225,539; and Winter, European Patent No. 0,239,400 B1. See also, Newman, R. et al., BioTechnology, 10: 1455-1460 (1992), regarding primatized antibody. See, e.g., Ladner et al., U.S. Pat. No. 4,946,778; and Bird, R. E. et al., Science, 242: 423-426 (1988)), regarding single chain antibodies.

In addition, a mixture of antibodies, termed herein as an "antibody preparation", can be used according to the methods of the present invention. Such antibody preparations include polyclonal and monoclonal mixtures of antibodies. A humanized antibody may comprise portions of immunoglobulins of different origin. For example, at least one portion can be of human origin. For example, the humanized antibody can comprise portions derived from an immunoglobulin of nonhuman origin with the requisite specificity, such as a mouse, and from immunoglobulin sequences of human origin (e.g., a chimeric immunoglobulin), joined together chemically by conventional techniques (e.g., synthetic) or prepared as a contiguous polypeptide using genetic engineering techniques (e.g., DNA encoding the protein portions of the chimeric antibody can be expressed to produce a contiguous polypeptide chain). Alternatively, a humanized antibody may be created in a transgenic or humanized animal expressing the human antibody genes (see Lonberg, N. "Transgenic Approaches to Human Monoclonal Antibodies." Handbook of Experimental Pharmacology 113 (1994): 49-101). Another example of a humanized antibody of the present invention is an immunoglobulin containing one or more immunoglobulin chains comprising a CDR of nonhuman origin (e.g., one or more CDRs derived from an antibody of nonhuman origin) and a framework region derived from a light and/or heavy chain of human origin (e.g., CDR-grafted antibodies with or without framework changes). Chimeric or CDR-grafted single chain antibodies are also encompassed by the term "humanized antibody".

Methods of preparing immunoglobulins, immunizing with antigens, and polyclonal and monoclonal antibody production can be performed as described herein, or using other suitable techniques. A variety of methods have been described. See e.g., Kohler et al., Nature, 256: 495-497 (1975) and Eur. J. Immunol. 6: 511-519 (1976); Milstein et al., Nature 266: 550-552 (1977); Koprowski et al., U.S. Pat. No. 4,172,124; Harlow, E. and D. Lane, 1988, Antibodies: A Laboratory Manual, (Cold Spring Harbor Laboratory: Cold Spring Harbor, N.Y.); Current Protocols In Molecular Biology, Vol. 2 (Supplement 27, Summer '94), Ausubel, F. M. et al., Eds., (John Wiley & Sons: New York, N.Y.), Chapter 11, (1991; Rasmussen SK, Rasmussen LK, Weilguny D, Tolstrup AB. Biotechnol Lett. 2007 Feb 20.). Polyclonal antibodies which may be used in the methods of the invention are heterogeneous populations of antibody molecules derived from the sera of immunized animals (using αVβ3-specific peptides or recombinant proteins as an immunogen). For preparation of Monoclonal antibodies directed to αVβ3, a hybridoma can be produced by fusing a suitable immortal cell line (e.g., a myeloma cell line such as SP2/0) with antibody producing cells. The antibody producing cell, preferably those of the spleen or lymph nodes, are obtained from animals immunized with the antigen of interest (αVβ3-specific peptide or recombinant protein). Hybridomas can be isolated using selective culture conditions, and cloned by limiting dilution. Cells that produce antibodies with the desired specificity can be selected by a suitable assay (e.g., ELISA). Antibodies can be purified from the plasma of human donors. This is the current approach for IVIg where immunoglobulins are isolated from normal plasma pooled from a large number of donors (see http://www.fda.gov/cber/gdlns/igivimmuno.htm and Haeney Clin Exp Immunol. 1994 July; 97(Suppl 1): 11-15.) Other suitable methods for producing or isolating antibodies of the requisite specificity can be used, including, for example, methods which select recombinant antibodies from a library, or which rely upon immunization of transgenic animals (e.g., mice) capable of producing a full repertoire of human antibodies. See e.g., Jakobovits et al., Proc. Natl. Acad. Sci. USA, 90: 2551-2555 (1993); Jakobovits et al., Nature, 362: 255-258 (1993); Lonberg et al., U.S. Pat. No. 5,545,806; Surani et al., U.S. Pat. No. 5,545,807.

An antibody may have one or more binding sites. If there is more than one binding site, the binding sites may be identical to one another or may be different. For instance, a naturally occurring immunoglobulin has two identical binding sites, a single-chain antibody or Fab fragment has one binding site, while a "bispecific" or "bifunctional" antibody has two different binding sites. An individual antibody's ability to internalize, deliver a payload, and kill a target cell can vary greatly depending on the affinity and the particular target epitope that is engaged by the antibody. For a given target in this case IL1RAPL-1 a panel of antibodies should be developed (will cover a range of binding affinities and epitopes) for ADC conjugation. The term "human antibody" includes all antibodies that have one or more variable and constant regions derived from human immunoglobulin sequences. In one embodiment, all of the variable and constant domains are derived from human immunoglobulin sequences (a fully human antibody). The term "chimeric antibody" refers to an antibody that contains one or more regions from one antibody and one or more regions from one or more other different antibodies. In one embodiment, one or more of the CDRs are derived from a specific human antibody. In a more preferred embodiment, all of the CDRs are derived from a human antibody. In another preferred embodiment, the CDRs from more than one human antibody are mixed and matched in a chimeric antibody. For instance, a chimeric antibody may comprise a CDR1 from the light chain of a first human antibody combined with CDR2 and CDR3 from the light chain of a second human antibody, and the CDRs from the heavy chain may be derived from a third antibody. Further, the framework regions may be derived from one of the same antibodies, from one or more different antibodies, such as a human antibody, or from a humanized antibody.

When used herein, the term "composition" should be understood to mean something made by the hand of man, and not including naturally occurring compositions. Compositions may be formulated in unit dosage form, i.e., in the form of discrete portions containing a unit dose, or a multiple or sub-unit of a unit dose.

When used herein, the term "pharmaceutical composition" may comprise one or more pharmaceutically acceptable diluents, excipients or carriers. Even though the peptides and compositions of the present invention can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients", 2nd Edition, (1994), edited by A Wade and PJ Weller. In particular, formulations for topical delivery are described in "Topical Drug Delivery Formulations" edited by David Osborne and Antonio Aman, Taylor & Francis, the complete contents of which are incorporated herein by reference. Acceptable carriers or diluents for therapeutic use are well known in the pharmaceutical art, and are described, for example, in Remington's Pharmaceutical Sciences, Mack Publishing Co. (A. R. Gennaro edit. 1985). Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, 20 magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of phydroxybenzoic acid. Antioxidants and suspending agents may be also used.

The term "derivative" means a compound differing from another compound by a structural modification, for example replacement of one atom or a group of atoms or a functional group with another atom or group of atoms or functional group.

When used herein "loss of barrier function" refers to impairment of normal barrier function. The barrier may be the endothelial or epithelial cell barrier in any part of the body.

The antagonist of the invention, or composition comprising same, may be administered individually or in combination with other pharmacologically active agents or additional ingredients. It will be understood that such combination therapy encompasses different therapeutic regimens, including, without limitation, administration of multiple agents together in a single dosage form or in distinct, individual dosage forms. If the agents are present in different dosage forms, administration may be simultaneous or near-simultaneous or may follow any predetermined regimen that encompasses administration of the different agents.

Such additional ingredients may be those of benefit to include in a composition, or of benefit depending on the intended use of the composition. The additional ingredient may be active or functional or both. If the agents are present in different dosage forms, administration may be simultaneous or near-simultaneous or may follow any predetermined regimen that encompasses administration of the different agents.

The additional ingredient may be one suitable for treatment of a respiratory or lung disease or a symptom thereof. The additional ingredient may be one suitable for treatment of ARDS or a symptom thereof, renal, or acute kidney injury (AKI), coagulopathies, cardiopathies, and/or neural pathologies. The additional ingredient may be one suitable for treatment of a disease with compromised barrier function. The additional ingredient may be an antiviral drug and/or an antibiotic. This may be called combination therapy.

Therapeutic agents suitable for diseases with compromised barrier function or conditions include but are not limited to anti-diarrhoeal agents, and agents against human or animal haemorrhagic viruses. It will be appreciated that such agents are known in the art and all are encompassed herein. The additional ingredient may be one suitable for treatment of pain and may be any suitable ingredient known in the art.

It is to be understood that additional ingredients listed may provide more than one benefit. The classification given herein is for clarity and convenience only and not intended to limit the additional ingredient to that particular application or category listed.

The nature and amount of any additional ingredient should not unacceptably alter the benefits of this invention. Typical said additional active agent is present in trace amounts only. In some embodiments, there may be no additional ingredient present in the composition. The amount of additional ingredient included will depend on numerous factors, including the type of additional ingredient used, the nature of the additional ingredient, the component(s) of the composition, the amount of ingredient or peptide in the composition and/or the intended use of the composition.

The additional ingredient may be an active agent It is to be understood that an ingredient that is considered to be an "active" ingredient in one product may be a "functional" or "excipient" ingredient in another and vice versa. It will also be appreciated that some ingredients play a dual role as both an active ingredient and as a functional or excipient ingredient.

Compositions also include compositions comprising any suitable combination of the antagonist of the invention and a suitable salt therefor. Any suitable salt, such as an alkaline earth metal salt in any suitable form (e.g., a buffer salt), can be used in the stabilization of the peptide of the invention (preferably the amount of salt is such that oxidation and/or precipitation of the peptide is avoided). Suitable salts typically include sodium chloride, sodium succinate, sodium sulfate, potassium chloride, magnesium chloride, magnesium sulfate, and calcium chloride. Compositions comprising a base and one or more peptides of the invention also are provided.

"Sepsis" is diagnosed when a patient exhibits at least two of the following symptoms, plus a probable or confirmed infection:
- Body temperature above 101 F (38.3 C) or below 96.8 F (36 C)
- Heart rate higher than 90 beats a minute
- Respiratory rate higher than 20 breaths a minute

Severe sepsis is diagnosed if the patient also exhibit at least one of the following signs and symptoms, which indicate an organ may be failing:
- Significantly decreased urine output
- Abrupt change in mental status
- Decrease in platelet count (thrombocytopenia)
- Difficulty breathing
- Abnormal heart pumping function
- Abdominal pain

Septic shock is diagnosed if in addition to the symptoms of severe sepsis the patient also displays extremely low blood pressure that does not adequately respond to simple fluid replacement.

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLES

### EXAMPLE 1

### METHODOLOGY

### Cell and virus culture conditions

The human colonic cell line (Caco-2) was maintained in DMEM High Glucose media supplemented with 10 % Fetal Bovine Serum, 1 % Penicillin and Streptomycin. Primary derived Human Aortic Endothelial Cells (HAoEC) were maintained in Endothelial Cell Media MV (PromoCell) supplemented with 10,000 U/mL Penicillin and 100 mg/mL Streptomycin. Cells were subject to shear hemodynamic force of 10 dynes/cm² to mimic the physiological conditions of vascular stress. SARS-CoV-2 (gift from N. Fletcher, University College Dublin, Ireland) was used throughout the duration of this study at a Multiplicity of Infection (MOI) of 2.5 unless otherwise stated.

### Para-Nitrophenyl Phosphate binding assay

SARS-CoV-2 interaction with Caco-2 and HAoEC was assessed using a binding assay utilising the fluorescent substrate, Para-Nitrophenyl Phosphate (pNPP). Inactivated virus aliquots were immobilised onto 96-well plates for 2 hours at 37 °C, followed by blocking with 1 % Bovine Serum Albumin (BSA). HAoEC were seeded at MOI of 8 for 2 hours at 37 °C. Treated cells were administered with 0.05, 0.005, and 0.0005 µM of the αVβ3 antagonist, Cilengitide, (gift from H. Kessler, Technical University of Munchen, Germany) for 1 hour. Cells were added onto immobilised SARS-CoV-2 and allowed to adhere for 2 hours. A lysis buffer (0.1M NaOAc, 15mM pNPP, 0.1% TritonX-100, pH=5.5) was added, containing the fluorescent marker para-nitrophenyl phosphate, which acts as a substrate for intracellular alkaline phosphatase. The reaction was stopped using 1M NaOH and pNPP activity was read at 405 nm in an automated plate reader (Victor, Perkins-Elmer). Following cell lysis, the resulting fluorescent signal was measured through absorbance at 405 nm. Binding (%) was analysed relative to the no drug control.

### Transwell permeability assay

Endothelial barrier injury of Caco-2 or sheared HAoEC was evaluated using a transwell permeability assay. Treated cells seeded onto top chambers received 0.0005µM Cilengitide for 1 hour and following SARS-CoV-2 infection for 24 hours, Fluorescein isothiocyanate-dextran (250ug/mL, 40kDa, Sigma-Aldrich) was added to the endothelial cells. Permeability was assessed by quantifying the levels of FITC-Dextran that had passed into the lower chamber. The fluorescent intensity was measured with excitation and emission wavelengths of 490 and 520nm, respectively.

### Immunofluorescence microscopy

VE-Cadherin (Vascular endothelial cadherin) expression on sheared HAoEC was measured by immunofluorescence microscopy. Sheared HAoEC were immobilised on glass slides, followed by SARS-CoV-2 infection for 24 hours. Cells were subsequently stained using anti-VE-Cadherin mouse monoclonal IgG1 antibody, conjugated to AlexaFluor 488 (SantaCruz Biotechnology), and 4,6-diamidino-2-phenylindole in fluorescent mounting medium (Invitrogen). Treated cells were subject to 0.0005µM Cilengitide for 1 hour. Images were acquired using an AxioObserver Z1 microscope (Zeiss). Levels of VE-Cadherin were computed by measuring fluorescent intensity of cells subtracted from background in ImageJ software (U. S. National Institutes of Health).

### Fluorescent-based protein interaction assay

Interactions between the SARS-CoV-2 Spike protein and endothelial αVβ3 were quantified using a fluorescent-based protein assay. Plates were coated with 30 µg/mL of Recombinant Human Novel Coronavirus Spike protein (Cusabio) for 2 hours at 37 °C. Plates were washed three times in PBS and blocked with 1 % BSA for 2 hours at 37 °C. Plates were washed as described, and 50 µg/mL of Recombinant Human Integrin alpha V beta 3 protein were added for 2 hours at 37 °C. After washing, plates were incubated with AlexaFluor 488 conjugated anti-Integrin αVβ3 clone LM609 diluted 1:100 in reagent diluent. After 1 hour, LM609 activity was measured at 450nm with an automated plate reader.

### Integrin-Spike protein structure modelling

The structures of SARS-CoV-2 spike protein and integrin αVβ3 in complex with RGD ligand were obtained from the RCSB Protein Data Bank (PDB ID: 6VXX and 1L5G, respectively). The RGD motif of the SARS-CoV-2 spike protein was aligned to the sequences of the RGD ligand in complex with αVβ3 to create a simulated model of interaction. All αVβ3-Spike protein structures and figures were visualised and prepared using Molecular Operating Environment (MOE) and PyMol.

### Statistical analysis

Data are presented as mean ± standard error of the mean. Experiments were carried out in triplicate with a minimum of three independent experiments. Statistical differences between groups were assessed by ANOVA with Dunnett's post hoc tests or t-tests, as indicated. A P-value of < 0.05 was considered to be significant.

### RESULTS AND DISCUSSION

The endothelium is at the forefront of microbial attack and plays a critical role in securing the integrity of the monolayer whilst maintaining an active response to stress. In response to injury induced by a pathogen, the delicate endothelium balance is disrupted, tipping towards vasculature dysregulation. This triggered response may go uninterrupted and become excessive in the presence of a severe infection, hence resulting in sepsis. The correlation between epithelial and endothelial damage during SARS-CoV-2 infection participation of the endothelium in SARS-CoV-2 infection remains unknown. However, the frequent occurrence of sepsis-like symptoms in COVID-19 patients such as elevated lactate dehydrogenase, D-dimers, and cytokine storms indicates sepsis is a shared outcome. In addition, 100 % of COVID-19 non-survivors develop sepsis, where they experience systemic inflammation, microthrombosis, acute cardiovascular complications, and severe organ dysfunction.

### Paracellular transport through human epithelial cells promotes entry of SARS-CoV-2 into the extracellular matrix

Histopathological data has confirmed SARS-CoV-2 actively targets and infects human epithelial cells, resulting in severe cytopathic effects. These primary manifestations commonly experienced in the lungs of COVID-19 patients include diffuse alveolar damage, tracheobronchitis, and interestingly, vascular injury. The current inventors investigated if SARS-CoV-2 is capable of gaining access to the endothelium, located below the epithelial layer and interstitial space. Although virus-induced apoptosis and cytokine upregulation are consistently documented *in-vitro,* perhaps an additional route exists by which SARS-CoV-2 could traverse across the epithelium. High expression of Tumor Necrosis Factor (TNF) has been widely reported in patients with COVID-19, where increased levels of this pro-inflammatory cytokine promotes intestinal epithelial barrier permeability. This disruption of intercellular junctions encourage a paracellular mechanism for viral invasion similar to that found in other viral infections such as Dengue virus and Human Immunodeficiency Virus (HIV).

The current inventors have demonstrated that SARS-CoV-2 binds strongly to epithelial cells (Fig. 1A) and engagement results in loss of epithelial barrier formation, significantly increasing over 48 hours of infection (Fig. 1B). This traversing facilitates dissemination of the virus into the interstitial space provides a mechanism through which it can gain entry to circulation and onward to all the major organs of the body.

### In-silico SARS-CoV-2 and endothelial αVβ3 complex reveals new potential binding pocket

Cumulative data suggests that the enhanced transmissibility and infectivity of SARS-CoV-2 is attributed to mutations on the spike protein. Sequence analysis reveals a novel mutation absent in all coronavirus predecessors (Fig. 2A,B). A point mutation at K403R introduces a widely recognised integrin recognition motif, Arginine-Glycine-Aspartic acid (RGD), into the spike protein. This region binds to the major human endothelial integrin, αVβ3, with high affinity. Strikingly, this evolutionary motif resides nearby but not within the ACE2 binding site. Therefore, alongside ACE2, the spike protein may also exploit a second human receptor. This would not only provide a more secure attachment onto host tissue, but also would ease SARS-CoV-2 dissemination between cells that simultaneously express both receptors.

To understand the structural interaction between SARS-CoV-2 and αVβ3, the inventors constructed an *in-silico* molecular simulation of the theoretical complex. The spike protein-ACE2 complex was compared to the αVβ3-RGD complex, both obtained from the RCSB Protein Data Bank. The inventors first examined the interaction between αVβ3 and its natural ligand, the RGD motif (Fig. 3A). Following this, the inventors located the novel RGD site within the spike protein of SARS-CoV-2 (Fig. 3B) and created the integrated virus-host complex by docking and aligning the RGD site of the spike protein into the αVβ3 structure (Fig. 3C). Interestingly, the RGD motif within the spike protein aligns perfectly to the ligand binding pocket of αVβ3, suggesting this interaction may occur during infection. The inventors then investigated the contrasting biochemical properties of SARS-CoV K390 and SARS-CoV-2 R403. Both lysine and arginine frequently appear on protein surfaces because of their critical role in maintaining ligand to receptor contacts through hydrogen and electrostatic bonds. The flexible glycine and bulky aspartic acid residues within the region of interest are shared in SARS-CoV and SARS-CoV-2 spike proteins (Fig. 2). However, the guanidinium group in arginine creates more interactions with αVβ3, due to 3 asymmetric amines, in contrast to lysine, which retains a lone amine group. This results in more electrostatic points of contact between SARS-CoV-2 and αVβ3 which moves the spike protein closer to its receptor. Furthermore, the high pKa of arginine offers more stable ionic bonds due to delocalization of the positive charge across the π bonds of guanidinium which results in more resonance forms. Therefore, in comparison to the lysine residue of SARS-CoV, the conservational replacement into arginine at site 403 is likely to be more favourable for αVβ3 interactions.

### SARS-CoV-2 interacts with human vascular endothelial cells through an evolutionary RGD mutation

Although ACE2 was identified as being the sole receptor involved with host attachment, entry and invasion, SARS-CoV-2 remains capable of targeting ACE2-negative colonic enterocytes and liver cells, which is particularly significant as acute hepatic impairment is prominent in COVID-19 patients. RNA expression of ACE2 indicate high levels within the alveolar epithelium and gastrointestinal tract. In contrast, the integrin αVβ3 retains cytoplasmic and surface membrane expression in nearly all tissues and cells of mesenchymal origin, including gastrointestinal, respiratory, and urinary systems. Additionally, αVβ3 has higher expression patterns across the alveolar and vascular endothelium than ACE2. This finding illustrates the substantial benefits that SARS-CoV-2 has attained by potentially gaining a second host receptor. Binding both ACE2 and αVβ3, which are respectively major epithelial and endothelial receptors, could explain how SARS-CoV-2 has expanded its tissue range of infection. The inventors investigated this matter by examining the interactions between purified and tagged spike protein and human endothelial αVβ3, and found SARS-CoV-2 spike protein binds strongly to αVβ3 (Fig 4A). Furthermore, the *ex-vivo* infection model indicated SARS-CoV-2 attaches to human vascular endothelial cells in the presence of activation marker, TNF-α. A remarkable finding revealed the spike protein-αVβ3 interaction can be blocked by the specific αVβ3-antagonist Cilengitide, a tripeptide compromised of Arg-Gly-Asp residues, in a dose-dependent manner (Fig 4B). The αVβ3 antagonistic molecule bound with high affinity to αVβ3, and was able to eliminate the interaction between the virus and the endothelium when used at 0.0005 µM.

### Vascular permeability and disruption of intercellular junctions induced by SARS-CoV-2 can be prevented by blocking host αVβ3 receptor

A primary manifestation of COVID-19 is the loss of endothelial barrier integrity, which promotes vascular leakage, tissue oedema, hypoxia, and also encourages the dissemination of the virus to all the major organs, contributing to multiple organ failure and ultimately, death. Vascular-endothelial cadherin (VE-cadherin) is a crucial tight junction protein that plays a key role in maintaining the intact endothelial layer. Addition of SARS-CoV-2 to human endothelial cells resulted in a significant increase in endothelial cell permeability suggesting a breakdown in barrier junctions (Fig. 5A). This was a significant finding, as changes in the endothelial morphology is likely accompanied by disruption of intercellular junctions and loss of contact as a whole monolayer. To investigate this the inventors measured the loss of VE-cadherin through quantitative immunofluorescence. Uninfected human endothelial cells maintained a tight barrier formation. However, upon infection with SARS-CoV-2, VE-cadherin surface expression on human endothelial cells was significantly reduced, suggesting cell-cell detachment and loss of barrier integrity occurred following infection (Fig. 5B,C). This likely occurred due to the activation of the β3 subunit through an RGD-containing ligand, here represented by the SARS-CoV-2 spike protein. Previous studies demonstrated that engagement of β3 results in VE-cadherin internalisation and increased vascular leakage. A striking observation was that Cilengitide was capable of inhibiting viral attachment to endothelial αVβ3, which in turn prevented VE-cadherin reduction during infection. It also limited the occurrence of barrier permeability, returning VE-cadherin levels back to uninfected levels (Fig. 5B,C).

In conclusion, SARS-CoV-2 binding to the epithelium creates a paracellular route of invasion by causing significant changes in permeability. Following this breakdown, the virus passes through the interstitial space and attaches to abluminally expressed aVb3 on the vascular endothelium. This interaction is likely mediated through the evolutionary K403R motif within the spike protein that promotes binding to αVβ3. This mutagenesis could elucidate the enigma of the endothelial role during COVID-19 pathogenesis, where vascular dysregulation is a prominent occurrence in severely affected patients. Additionally, a critical observation is that in all major SARS-CoV-2 variants, including the Clustal 5 and VUI 202012/01 variants, the RGD site is conserved, suggesting these coronaviruses maintain the ability to anchor to αVβ3. This contrasts all coronavirus predecessors that lack the RGD motif. A remarkable finding of the inventors' work has identified that the addition of an αVβ3 antagonist, Cilengitide, significantly prevents both attachment to the host and disruption of barrier integrity.

### EXAMPLE 2

### MATERIALS AND METHODS

### Cell and virus culture conditions

The human colonic cell line (Caco-2; ATCC^{®} HTB-37) were maintained in DMEM High Glucose media supplemented with 10% Fetal Bovine Serum, 1% Penicillin and Streptomycin. Primary derived Human Aortic Endothelial Cells (HAoEC; Promocell C-12271) were maintained in Endothelial Cell Media MV (PromoCell) supplemented with 10,000 U/mL Penicillin and 100 mg/mL Streptomycin. HAoEC were subject to shear hemodynamic force of 10 dynes/cm2 to mimic the physiological conditions of vascular stress. Human 2019-nCoV strain 2019-nCoV/ltaly-INMI1 was obtained from the European Virus Archive Global (Ref. no: 008V-03893) and was used throughout the duration of this study at a Multiplicity of Infection (MOI) of 0.4 with viral titer at 1.08 x 10⁵ TCID50/mL.

### Para-nitrophenyl phosphate infection model

SARS-CoV-2 interaction with Caco-2 and HAoEC was assessed using a binding assay utilising the fluorescent substrate, Para-Nitrophenyl Phosphate (pNPP). Inactivated virus aliquots were immobilised onto 96-well plates for 2 hours at 37°C, followed by blocking with 1% Bovine Serum Albumin (BSA). Cells were seeded for 2 hours at 37°C. Treated cells were administered with 0.05, 0.005, and 0.0005 µM of the αVβ3 antagonist, Cilengitide, (gift from H. Kessler, Technical University of Munchen, Germany) for 1 hour. Cells were also administered with GLPG0187 (MedChemExpress) or SB-273005 (SelleckChem) under similar conditions. Cells were added onto immobilised SARS-CoV-2 and allowed to adhere for 2 hours. A lysis buffer (0.1M NaOAc, 15mM pNPP, 0.1% TritonX-100, pH = 5.5) was added, containing the fluorescent marker para-nitrophenyl phosphate, which acts as a substrate for intracellular alkaline phosphatase. The reaction was stopped using 1M NaOH and pNPP activity was read at 405 nm in an automated plate reader (Victor, Perkins-Elmer). Following cell lysis, the resulting fluorescent signal was measured through absorbance at 405 nm. Binding (%) was analysed relative to the untreated cells.

### Immunofluorescence microscopy

VE-Cadherin (Vascular endothelial cadherin) expression on sheared HAoEC was measured by immunofluorescence microscopy. Sheared HAoEC were immobilised on glass slides, followed by SARS-CoV-2 infection for 24 hours. Cells were subsequently stained using anti-VE-Cadherin mouse monoclonal IgG1 antibody, conjugated to AlexaFluor 488 (1:100, F-8 sc-9989, SantaCruz Biotechnology), and 4,6-diamidino-2-phenylindole in fluorescent mounting medium (Invitrogen). Treated cells were subject to 0.0005µM Cilengitide for 1 hour. To measure internalised VE-Cadherin, cells were briefly acid washed (25 mM glycine, 3 % BSA at pH=2.7) following antibody incubation. Cells were then fixed in 4 % formaldehyde, permeabilized, and loaded onto a DAPI-coated glass slide for processing. Images were acquired using an AxioObserver Z1 microscope (Zeiss). Levels of VE-Cadherin were computed by measuring fluorescent intensity of cells subtracted from background in ImageJ software (U. S. National Institutes of Health).

### SDS-PAGE and Western blot

Following the ex vivo infection assay, endothelial cells were lysed in RIPA buffer and stored at -20 °C until use. Samples were separated on a 10 % SDS-PAGE gel. Following this, proteins were transferred onto a nitrocellulose membrane and blocked for 1 hour using 5 % skimmed milk. The membrane was probed using anti-VE Cadherin primary antibody (Clone: F-8, Cat. No: sc-9989, 1:200, SantaCruz) and anti-GAPDH primary antibody (6000-4-1 mouse mAB, 1:2000, ProteinTech), overnight at 4 °C. Anti-mouse IgG secondary antibodies (sc525405, 1:5000, SantaCruz) were used the following day. Proteins were detected using chemiluminescence.

### Fluorescent-based protein interaction assay

Interactions between the SARS-CoV-2 Spike protein and endothelial αVβ3 were quantified using a fluorescent-based protein assay. Plates were coated with 25 ng of Recombinant integrin αVβ3 (Cat. No: 3050-AV-050, BioTechne) overnight at 4°C. Plates were washed three times in PBS and blocked with 1% BSA for 2 hours at 37°C. Plates were washed as described. Anti-β3 (Clone: B-7, Cat. No: SC-46655, SantaCruz) and anti-αVβ3 (Clone: MAB1976, Sigma) monoclonal antibodies were added (5 µg/mL) in reagent diluent for 1 hour at room temperature to test direct integrin subunit involvement during spike protein - integrin recognition. Following this, recombinant spike protein (Cat. No: CSB-MP3324GMYSP, Cusabio) was added at 50 nM for 1 hour at room temperature. Anti-spike protein conjugated to AlexaFluor 405 (Clone: 1035206, BioTechne) was added at 1:100 for 1 hour in the dark. Samples were read using an automated plate reader at absorbance 405 nm.

### Molecular modelling

The Cryo-EM 3D structure of SARS-CoV-2 spike protein (Delta V.O.C.) and wild-type were obtained from the RCSB Protein Data Bank (PDB ID:7V8A and 7KNB). The wild-type spike protein underwent substitution mutagenesis using the Protein Builder tool in MOE to create a model of the SARS-CoV-2 Omicron V.O.C. FASTA sequences were retrieved from NCBI and aligned using ClustalOmega multiple sequence alignment tool. The AMBER10:EHT force field in MOE was used to energy minimise the complexes to resolve the intramolecular interactions and remove modelling bias before and after docking. The SCWRL algorithm and AMBER2 force field were used to obtain binding affinity through the molecular dynamics simulation function in Yasara. All structures and figures were visualised and prepared using Molecular Operating Environment (MOE) and PyMol.

### RESULTS AND DISCUSSION

### Effect of cilengitide on SARS-CoV-2 binding to human colorectal epithelial cells

As shown in Figure 7, cilengitide (0.005µM and 0.05µM) effectively reduces SARS-CoV-2 binding to human colorectal epithelial cells in an in vitro infection model.

### Cilengitide prevents binding between the spike protein of SARS-CoV-2 and αVβ3

The inventors have shown that both αVβ3 and β3 antibodies can inhibit spike protein binding to αVβ3, revealing significant interactions between the receptors. Cilengitide prevents binding between recombinant spike protein and αVβ3 at 0.0005 µM. This is illustrated in Figure 8.

### Attachment of SARS-CoV-2 to human vascular endothelial cells in the presence of inhibitors GLPG0187 and SB273005 and cilengitide.

The results show that αVβ3 integrin inhibitors GLPG0187 and SB273005 failed to inhibit viral (SARS-CoV-2) attachment to human vascular endothelial cells at a range of concentrations (5, 0.05, 0.005 mM). These results are shown in Figure 9.

In contrast to this finding, cilengitide dose dependently inhibits virus (SARS-CoV-2) attachment to human vascular endothelial cells at a range of concentrations (0.05, 0.005, 0.0005 mM).

These data suggest that cilengitide is behaving in a different unexpected beneficial way in preventing viral attachment to human vascular endothelial cells in comparison to other integrin antagonists.

### Cilengitide prevents VE-cadherin internalisation following SARS-CoV-2 infection.

Following infection by SARS-CoV-2, the vascular endothelium experiences severe monolayer dysfunction, identified by VE-Cadherin relocation from the external cell-cell contacts to internal cellular compartments. The internalisation of VE-Cadherin is prevented in the presence of Cilengitide, in an in vitro model of infection (0.0005 µM). Total VE-Cadherin remains consistent in both healthy, infected, and treated cells, revealing VE-Cadherin translocation patterns (Figure 10).

### In silico modelling

SARS-CoV-2 variants of concern Alpha, Beta, Gamma, Delta and Omicron spike proteins aligned to show conservation of RGD site at 403-405 (Figure 11). *In silico* modelling of Omicron and Delta spike proteins reveals RGD motif of Omicron variant extends outwards to the solvent more than Delta.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. Cilengitide for use in a method of treatment or prevention of a coronavirus infection in a subject.

2. Cilengitide for use of Claim 1, wherein the coronavirus is one comprising an RGD domain in the receptor binding domain of the spike protein.

3. Cilengitide for use of Claim 1 or 2, wherein the coronavirus is SARS-CoV-2 and the subject has COVID-19.

4. Cilengitide for use of any one of the preceding claims, which binds to αVβ3 integrin on vascular endothelial cells.

5. Cilengitide for use of any one of the preceding claims, wherein said cilengitide is to be administered at a dosage of from 0.001 to 10mg/m².

6. Cilengitide for use of Claim 5, wherein the dosage to be administered is from 0.001 to 1mg/m².

7. Cilengitide for use of any one of the preceding claims, by early treatment or prevention of loss of barrier function associated with the infection.

8. Cilengitide for use in the treatment or prevention of cardiovascular dysfunction associated with a coronavirus infection.

9. Cilengitide for use of any one of the preceding claims, which is formulated as a composition comprising cilengitide optionally comprising at least one pharmaceutically acceptable carrier or excipient.

10. Cilengitide for use of Claim 9, wherein the composition is formulated for pulmonary delivery or intravenous delivery.

11. Cilengitide for use of Claim 9, wherein the composition is formulated for oral delivery.

12. Cilengitide for use of any one of the preceding claims, wherein said cilengitide, is to be administered with one or more respiratory or lung disease treatments.

13. Cilengitide for use in a method of treatment or prevention of coronavirus disease **characterised by** loss of barrier function in a subject.

14. Cilengitide for use of Claim 13, wherein the coronavirus is SARS-CoV-2 and the subject has COVID-19.

## Patentansprüche

1. Cilengitid für die Verwendung in einem Verfahren zur Behandlung oder Prävention einer Coronavirus-Infektion bei einem Subjekt.

2. Cilengitid für die Verwendung nach Anspruch 1, wobei das Coronavirus eines ist, das eine RGD-Domäne in der Rezeptor-Bindungsdomäne des Spikes-Proteins umfasst.

3. Cilengitid für die Verwendung nach Anspruch 1 oder 2, wobei das Coronavirus SARS-CoV-2 ist und das Subjekt COVID-19 hat.

4. Cilengitid für die Verwendung nach einem der vorstehenden Ansprüche, das zum αVβ3-Integrin auf vaskulären Endothelzellen bindet.

5. Cilengitid für die Verwendung nach einem der vorstehenden Ansprüche, wobei das Cilengitid in einer Dosierung von 0,001 bis 10 mg/m² zu verabreichen ist.

6. Cilengitid für die Verwendung nach Anspruch 5, wobei die zu verabreichende Dosierung von 0,001 bis 1 mg/m² beträgt.

7. Cilengitid für die Verwendung nach einem der vorstehenden Ansprüche durch frühzeitige Behandlung oder Prävention des Verlustes von Barrierefunktion, der mit der Infektion verbunden ist.

8. Cilengitid für die Verwendung bei der Behandlung oder Prävention von kardiovaskulärer Funktionsstörung, die mit einer Coronavirus-Infektion verbunden ist.

9. Cilengitid für die Verwendung nach einem der vorstehenden Ansprüche, das als eine Zusammensetzung formuliert ist, die Cilengitid umfasst, optional mindestens einen pharmazeutisch verträglichen Träger oder Hilfsstoff umfasst.

10. Cilengitid für die Verwendung nach Anspruch 9, wobei die Zusammensetzung zur pulmonalen Abgabe oder intravenösen Abgabe formuliert ist.

11. Cilengitid für die Verwendung nach Anspruch 9, wobei die Zusammensetzung zur oralen Abgabe formuliert ist.

12. Cilengitid für die Verwendung nach einem der vorstehenden Ansprüche, wobei das Cilengitid mit einer oder mehreren Behandlungen von Atemwegs- oder Lungenerkrankung zu verabreichen ist.

13. Cilengitid für die Verwendung in einem Verfahren zur Behandlung oder Prävention von Coronavirus-Erkrankung, die durch Verlust von Barrierefunktion gekennzeichnet ist, bei einem Subjekt.

14. Cilengitid für die Verwendung nach Anspruch 13, wobei das Coronavirus SARS-CoV-2 ist und das Subjekt COVID-19 hat.

## Revendications

1. Cilengitide pour une utilisation dans un méthode de traitement ou de prévention d'une infection par coronavirus chez un sujet.

2. Cilengitide pour une utilisation selon la revendication 1, le coronavirus étant un coronavirus comprenant un domaine RGD dans le domaine de liaison au récepteur de la protéine Spike.

3. Cilengitide pour une utilisation selon la revendication 1 ou 2, le coronavirus étant le SARS-CoV-2 et le sujet étant atteint de la COVID-19.

4. Cilengitide pour une utilisation selon l'une quelconque des revendications précédentes, qui se lie à l'intégrine αVβ3 sur les cellules endothéliales vasculaires.

5. Cilengitide pour une utilisation selon l'une quelconque des revendications précédentes, ledit cilengitide devant être administré selon un dosage allant de 0,001 à 10 mg/m².

6. Cilengitide pour une utilisation selon la revendication 5, le dosage à administrer allant de 0,001 à 1 mg/m².

7. Cilengitide pour une utilisation selon l'une quelconque des revendications précédentes, par le traitement précoce ou la prévention de la perte de la fonction barrière associée à l'infection.

8. Cilengitide pour une utilisation dans le traitement ou la prévention d'un dysfonctionnement cardiovasculaire associé à une infection par coronavirus.

9. Cilengitide pour une utilisation selon l'une quelconque des revendications précédentes, qui est formulé sous la forme d'une composition comprenant du cilengitide, comprenant éventuellement au moins un support ou excipient pharmaceutiquement acceptable.

10. Cilengitide pour une utilisation selon la revendication 9, la composition étant formulée pour une administration par voie pulmonaire ou une administration par voie intraveineuse.

11. Cilengitide pour une utilisation selon la revendication 9, la composition étant formulée pour une administration par voie orale.

12. Cilengitide pour une utilisation selon l'une quelconque des revendications précédentes, ledit cilengitide devant être administré avec un ou plusieurs traitements contre les maladies respiratoires ou pulmonaires.

13. Cilengitide pour une utilisation dans une méthode de traitement ou de prévention d'une maladie à coronavirus **caractérisée par** une perte de la fonction barrière chez un sujet.

14. Cilengitide pour une utilisation selon la revendication 13, le coronavirus étant le SARS-CoV-2 et le sujet étant atteint de la COVID-19.
